# FASCICULE DE BREVET EUROPEEN

(11) **EP 3 697 528 B1**
(45) Date de publication et mention de la délivrance du brevet: **18.12.2024**
(21) Numéro de dépôt: 18803734.5
(22) Date de dépôt: 12.10.2018
(51) Int. Cl.: B01J 19/00

(54) **CASSETTE MICROFLUIDIQUE DE SYNTHÈSE D'UN RADIO-TRACEUR ET PROCÉDÉ DE SYNTHÈSE D'UN RADIO-TRACEUR AVEC UNE TELLE CASSETTE**
MIKROFLUIDISCHE KASSETTE ZUR SYNTHESE EINES RADIOTRACERS UND VERFAHREN ZUR SYNTHESE EINES RADIOTRACERS MIT SOLCH EINER KASSETTE
MICROFLUIDIC CASSETTE FOR SYNTHESISING A RADIOTRACER AND METHOD FOR SYNTHESISING A RADIO-TRACER WITH SUCH A CASSETTE

(30) Priorité: 18.10.2017 FR 1759802
(43) Date de publication de la demande: 26.08.2020
(73) Titulaire: P M B, 13790 Peynier (FR)
(72) Inventeur: HOURTANE, Virginie, La Corneirelle 13790 PEYNIER (FR); TANGUY, Laurent, La Corneirelle 13790 PEYNIER (FR); PINEDA, Florian, 13100 Aix En Provence (FR)
(74) Mandataire: Santarelli
(86) Numéro de dépôt international: PCT/FR2018/052540
(87) Numéro de publication internationale: WO 2019/077238

(56) Documents cités:
- WO-A1-2011/037615
- WO-A1-2013/101568
- WO-A1-2015/101539
- WO-A2-03/078358
- US-A1- 2011 150 714
- US-A1- 2015 157 743

## Description

La présente demande concerne une cassette microfluidique de synthèse d'un radio-traceur.

Une cassette de radiochimie actuelle repose sur l'utilisation de différents composants macroscopiques du commerce, à savoir par exemple des vannes mécaniques, des colonnes de billes, des fioles, etc., qui sont assemblés les uns aux autres pour permettre la réalisation de radio-synthèses, c'est-à-dire de synthèses d'un radio-traceur, une molécule radio-pharmaceutique associant une molécule d'intérêt pharmaceutique ou médical à un radio-isotope.

Une telle cassette est principalement destinée à la production d'une grande quantité d'un seul produit qui est ensuite distribué à plusieurs hôpitaux ou centres de soin par exemple.

Elle a en outre un volume total imposant. Les différents composants ne sont pas optimisés pour produire des faibles quantités de radiotraceurs, par exemple pour un seul patient.

Par conséquent, ils conduisent à utiliser une quantité excessive de réactifs et radio-isotopes pour favoriser le résultat de la réaction, c'est-à-dire la synthèse d'un radio-traceur voulu. De plus, de tels composants ont d'importants volumes morts et leur taille implique qu'une forte radioactivité demeure dans la cassette après la synthèse, interdisant une manipulation de la cassette par un opérateur avant de nombreuses heures, en fonction de la demi-vie du radio-isotope utilisé.

Enfin, une telle cassette constitue ensuite un déchet radioactif volumineux.

La demande WO 2016/166486 décrit par exemple une cassette permettant de résoudre, au moins en partie, les inconvénients précités, en menant en outre à d'autres avantages. WO2015/101539 décrit une cassette nicrofluidique de synthèse d'un radio-traceur.

Ainsi, au moins un des objectifs de la présente demande est d'améliorer une telle cassette, en menant en outre à d'autres avantages.

A cet effet, est proposé selon un premier aspect, une cassette microfluidique de synthèse d'un radio-traceur comportant :
- Une carte de support,
- Un circuit microfluidique, intégré au moins en partie dans la carte de support, comportant :
   ∘ au moins une prise d'alimentation par une fiole, configurée pour raccorder une fiole au circuit microfluidique,
   ∘ au moins un port isotope, configuré pour introduire un radio-isotope dans le circuit microfluidique,
   ∘ au moins une chambre de réaction, raccordée à l'au moins une prise d'alimentation par une fiole et à l'au moins un port isotope par des capillaires,
   ∘ au moins une chambre de mélange, positionnée en amont de l'au moins une chambre de réaction et raccordée à l'au moins une chambre de réaction en amont de laquelle elle est positionnée par au moins un capillaire,
   ∘ au moins une chambre de formulation, raccordée à l'au moins un port isotope et à l'au moins une prise d'alimentation par une fiole et positionnée en aval de l'au moins une chambre de réaction, et
   ∘ au moins une prise de connexion pour une seringue, positionnée en aval de l'au moins une chambre de formulation et raccordée à l'au moins une chambre de formulation par au moins un capillaire ; dans le cadre de la présente demande, une seringue désigne tout récipient permettant de récupérer le radio-traceur obtenu en sortie de la cassette ; cette dénomination inclut par exemple ici un réservoir ou un flacon.

Une telle carte de support mesure typiquement de l'ordre de la dizaine de centimètres de côté, par exemple entre 10 cm et 30 cm de côté, et moins de 7 cm d'épaisseur, voire moins de 5 cm d'épaisseur.

En d'autres termes, la cassette comporte principalement :
- une partie dite « microfluidique », composée en partie de capillaires, i.e. de canaux de faibles dimensions (typiquement d'environ 500 µm (micromètres) de diamètre ou de côté), de chambres de réaction (chacune d'un volume de l'ordre du microlitre, par exemple compris entre 10µL et 500µL), de chambres de mélange et d'une chambre de formulation du produit final (d'un volume de l'ordre du millilitre), et
- une partie configurée pour recevoir au moins une fiole, de préférence plusieurs fioles, par exemple entre 2 et 10 fioles.

L'architecture interne, fluidique, de la cassette est basée sur une décomposition des radio-synthèses en étapes successives.

En effet, pour miniaturiser un dispositif de synthèse d'un radio-traceur, les étapes habituelles de synthèse en termes de volume, débit, chimie ont dû être réorganisées et adaptées. Une autre difficulté a été d'éviter des apparitions de bulles. Autrement dit, une difficulté à « miniaturiser » un dispositif de synthèse d'un radio-traceur était notamment d'éviter des apparitions de bulles tout en permettant de mélanger ensemble différents réactifs nécessaires, et ce dans des capillaires et des chambres qui mesurent de l'ordre du micromètre.

Ainsi, bien qu'une telle cassette constitue un déchet radioactif, en particulier dans le cas du fluor et du gallium dont les impuretés radioactives ont une très longue durée de vie, elle permet une minimisation des quantités de fluides utilisées et donc une diminution des déchets générés et de la radioactivité résiduelle après la synthèse, principalement du fait de sa compacité.

La chambre de formulation se distingue principalement par son volume plus important que les autres chambres.

Dans un exemple de réalisation, la chambre de formulation comporte un port, par exemple situé en partie supérieure de la chambre de formulation, configuré pour permettre d'évacuer du gaz lors d'une étape de remplissage de la chambre de formulation.

De plus, par exemple, elle permet de refaire une étape de mélange entre une solution de NaCl et une molécule radio-pharmaceutique.

Dans un exemple privilégié de réalisation, en amont d'au moins une chambre de réaction se trouve au moins une structure configurée pour mélanger au moins deux fluides, il s'agit d'au moins une chambre de mélange.

Par exemple, au moins une chambre de mélange est configurée pour mélanger un précurseur, provenant d'une fiole, au radio-isotope et/ou pour diminuer une concentration de CH₃CN (acétonitrile) en sortie d'une colonne HPLC (acronyme de « High Performance Liquid Chromatography »), par exemple avant un changement de solvant.

Par exemple, le précurseur est stocké dans une des fioles. Le précurseur va réagir avec le radio-isotope pour former un radio-traceur, voire un radio-pharmaceutique injectable à un individu.

Par exemple, l'au moins une chambre de mélange comporte un capillaire et le capillaire comporte une paroi dont au moins une partie comporte une structure en relief.

Une telle structure en relief, ou microstructure, est ainsi configurée pour créer des turbulences, par exemple des vortex, dans des écoulements de fluides et ainsi augmenter fortement un mélange entre les fluides.

Une chambre de réaction est ici par exemple une cavité formée par un élargissement d'un tronçon d'un capillaire.

Dans un exemple de réalisation privilégié, l'au moins une chambre de réaction est une chambre de réaction en température, c'est-à-dire pour une réaction à une température différente de la température ambiante.

La chambre de réaction en température permet par exemple de faire réagir le précurseur avec le radio-isotope, à des températures différentes de la température ambiante si nécessaire.

Par exemple, la chambre de réaction en température est chauffée, c'est-à-dire que la température est portée à une température supérieure à la température ambiante.

Selon un autre exemple, il est également possible de refroidir la chambre de réaction en température, i.e. que la température est portée à une température inférieure à la température ambiante.

Par exemple, elle est refroidie après une réaction pour pouvoir transférer le liquide aux étapes suivantes, et/ou par exemple pour améliorer le rendement de certaines opérations de chimie (par exemple un piégeage de molécule).

A cet effet, par exemple, un circuit de chauffage comporte un élément chauffant/refroidissant, c'est-à-dire un élément configuré pour chauffer et refroidir, situé par exemple sur une plateforme de réception de la carte de support.

En effet, grâce à une telle cassette microfluidique, c'est-à-dire un dispositif miniaturisé, il est alors possible d'utiliser un même élément pour chauffer ou refroidir une chambre ; ce qui n'est pas possible pour des volumes plus importants.

En position, la chambre de réaction en température est ainsi en contact avec l'élément chauffant/refroidissant par exemple.

Dans un autre exemple de réalisation privilégié, la cassette comporte au moins deux chambres de réaction dont une est une chambre de réaction en température et une autre est une chambre de réaction alors dite « classique ».

Si la cassette comporte une chambre de réaction en température, la carte de support comporte en outre de préférence un circuit d'isolation thermique entourant au moins une partie de la chambre de réaction en température.

Dans un exemple de réalisation, le circuit d'isolation thermique comporte au moins un évidement traversant une épaisseur de la carte de support et s'étendant autour d'au moins une partie de la chambre de réaction en température.

Autrement dit, la chambre de réaction en température est de préférence physiquement séparée du reste de la carte, par exemple grâce à une ouverture découpée dans la carte, permettant ainsi d'éviter de chauffer, ou refroidir, des éléments autour par conduction thermique.

Ainsi, la cassette peut comporter une chambre de réaction en température même si elle n'est pas utilisée, ou n'est pas utilisée en température, lors d'un procédé de synthèse d'un radio-traceur.

Par exemple, pour certaines synthèses de radio-traceur à partir de carbone 11 sous état gazeux, il est possible de ne pas chauffer.

Dans un exemple de réalisation, la carte comporte en outre au moins un évent en amont et/ou un évent en aval de la chambre de réaction en température pour évacuer des gaz.

Par exemple, un évent est contrôlable entre une position ouverte et une position fermée.

Selon un autre exemple, un évent comporte une membrane poreuse qui permet d'évacuer des gaz vers le port déchet, notamment quand des vannes sont ouvertes.

Dans un exemple de réalisation privilégié, l'au moins une chambre de réaction est chargée en billes, en d'autres termes elle comporte une colonne de billes.

En particulier, il s'agit de préférence d'une chambre de réaction classique.

Par exemple, selon le radio-traceur à synthétiser, toutes les chambres de réaction sont chargées en billes ; et de préférence les chambres de réactions classiques.

De préférence, une chambre de réaction en température est dépourvue de billes.

De préférence, une chambre de réaction en température est vide avant utilisation de la cassette et n'est remplie que par des réactifs et fluides correspondants au cours de son utilisation.

Les billes sont par exemple intégrées lors de la fabrication de la carte et sont choisies en fonction des besoins, par exemple en fonction de la synthèse souhaitée et par conséquent en fonction du radio-isotope, pour un changement de solvant, une pré-purification, une purification, etc.

Ainsi les billes sont possiblement de natures différentes selon la chambre de réaction dans laquelle elles sont embarquées.

Ce sont par exemple des billes de QMA (quaternary ammonium anion exchange), qui favorisent une capture d'anions, ou des billes d'alumine, qui favorisent une pré-purification de radio-traceurs fluorés.

De préférence, les billes ont un diamètre d'au moins 25 µm (micromètre), voire d'au moins 30 µm.

Dans un exemple de réalisation intéressant, la cassette comporte en outre un support de fiole. Par exemple, le support de fiole est fixé, par exemple scellé, sur la carte de support. Le support de fiole comporte par exemple au moins un emplacement configuré pour recevoir une fiole où débouche l'au moins une prise d'alimentation par une fiole.

Ainsi, en utilisation par exemple, au moins une fiole est montée sur le support de fiole à un emplacement et est raccordée à l'au moins une prise d'alimentation par une fiole du circuit microfluidique.

Hormis le radio-isotope acheminé par le port isotope, tous les réactifs sont apportés par des fioles.

La carte de support peut donc ne contenir aucun réactif embarqué, hormis les billes éventuellement.

La cassette est par exemple configurée pour comporter au moins une fiole, et de préférence plusieurs fioles, par exemple entre 2 et 10 fioles.

Par exemple, parmi les emplacements configurés pour recevoir une fiole, au moins un emplacement est configuré pour recevoir une fiole de type 4R (par exemple ISO8362, une fiole 4R a une capacité de 6 mL (millilitre) mais elle est généralement remplie jusqu'à 4 mL maximum) et/ou un emplacement est configuré pour recevoir une fiole de type 15R (une fiole 15R a une capacité de 19 mL (millilitre) mais elle est généralement remplie jusqu'à 15 mL maximum).

L'utilisation et/ou l'emplacement des différentes fioles sont à adapter en fonction de la synthèse à réaliser. Néanmoins certaines sont fréquemment utilisées, comme par exemple une fiole d'eau déminéralisée (« DI water »), une fiole de précurseur, ou encore une fiole de chlorure de sodium (NaCl).

Optionnellement, la cassette, ou en particulier le circuit microfluidique, comporte en outre au moins un port gaz configuré pour injecter du gaz dans l'au moins une fiole.

Ainsi la pression de gaz injectée par le port gaz, via un capillaire, permet de chasser un fluide contenu dans la fiole par un autre capillaire, c'est-à-dire par la prise d'alimentation par une fiole.

Le capillaire du port gaz débouche donc d'un côté à un emplacement configuré pour recevoir une fiole du support de fiole.

Dans un exemple de réalisation, la cassette comporte au moins une vanne, de préférence plusieurs vannes, permettant le pilotage des fluides en provenance des différentes interfaces, c'est-à-dire des différentes fioles et/ou du port isotope par exemple.

Par exemple, le circuit microfluidique comporte au moins une vanne configurée pour ouvrir/fermer un capillaire.

La cassette est par exemple possiblement reliée à un circuit pneumatique et une commande de contrôle est configurée pour piloter l'au moins une vanne en ouverture/fermeture.

L'au moins une vanne est pilotée individuellement, c'est-à-dire qu'en cas de pluralité de vanne chaque vanne est pilotée unitairement ; par exemple par énergie pneumatique ou mécanique.

Par exemple, chaque vanne est pilotée par l'intermédiaire d'une commande de contrôle.

L'au moins une vanne est actionnée à une pression comprise entre environ 0 et 7 bars mais de préférence autour de 3-4 bars.

Elles ont un très faible volume mort et sont activées/désactivées en moins d'une seconde. Une telle vanne assure également un taux de fuite extrêmement faible, même lorsqu'elle est utilisée pour la chambre de réaction en température ; à titre d'exemple la chambre de réaction en température peut subir une pression interne de quelques bars lors d'un chauffage des solvants à 130 °C.

Une telle vanne est par exemple formée par une membrane plastique configurée pour fermer un capillaire lorsqu'une pression lui est appliquée.

Ainsi, le nombre de vannes activées et/ou le choix de fioles, leur nombre, leur volume et leur contenu, permettent une certaine flexibilité dans l'enchainement des réactions et des étapes de synthèse.

Selon encore un autre exemple, la cassette comporte en outre un détrompeur configuré pour positionner la cassette.

Dans un exemple de réalisation particulier, un tel détrompeur est par exemple positionné de côté de la carte, i.e. sur un pourtour de la carte.

Un tel détrompeur désigne par exemple ici des structures qui permettent à un robot de se saisir avec précision de la cassette pour la manipuler dans un espace automatisé et/ou différentes structures mécaniques permettant de positionner et/ou d'aligner la cassette avec un équipement associé. Un tel environnement comportant un robot et un équipement associé est par exemple décrit dans la demande WO 2016/166486.

En outre, une telle cassette est configurée pour être raccordée à :
- Une colonne HPLC pour une étape de purification qui, du fait des exigences techniques et chimiques, est une étape peu facilement intégrable au sein du circuit microfluidique, et encore moins pour de multiples synthèses ; et/ou
- Un filtre stérilisant, pour une étape de filtration stérilisante : une telle étape est nécessaire pour préparer le produit à injecter à un individu, cette étape, pour des raisons réglementaires et techniques est plus facile à maintenir à l'extérieur de la cassette. Un mode de réalisation envisagé de la cassette permet toutefois l'intégration d'un tel filtre.

Ainsi, selon un autre exemple de réalisation, la carte de support comporte aussi un filtre stérilisant et un port contrôle-qualité servant à réaliser un test en sortie du filtre stérilisant. Par exemple le filtre stérilisant est positionné entre la chambre de formulation et le port seringue ; le port contrôle-qualité est par exemple positionné en parallèle du port seringue, en aval du filtre stérilisant.

Des modes de mise en oeuvre privilégiés utilisent principalement trois radio-isotopes différents, à savoir :
- ¹⁸F (fluor 18) en solution dans de l'eau enrichie en ¹⁸O (oxygène 18), cette solution est par exemple une cible de cyclotron,
- ¹¹C (carbone 11), soit sous la forme iodométhane (CH₃I) gazeux, poussé par de l'Hélium (He), soit CH₃I liquide dissout dans du DMSO (diméthylsulfoxyde) ou DMF (diméthylformamide), soit CHsOTf (triflate de méthyle) gazeux, soit sous la forme de monoxyde de carbone CO, soit sous la forme de dioxyde de carbone CO₂.
- ⁶⁸Ga (gallium 68) en solution dans de l'eau par exemple enrichie en ⁶⁸Zn (zinc 68), cette solution est par exemple une cible de cyclotron ; ou une solution de ⁶⁸Ga issue d'un générateur de gallium 68.

Toutefois, d'autres radio-isotopes peuvent être utilisés comme par exemple des radio-isotopes dédiés à de la radiothérapie vectorisée, comme par exemple du 90-Yttrium, 177-Astate, 212-Plomb ou autres.

Ainsi, une telle cassette permet de synthétiser différents radio-traceurs à partir d'une même architecture. En sortie de la cassette, il est possible de filtrer le radio-traceur avec un filtre stérilisant afin d'obtenir un radio-pharmaceutique injectable à un individu.

Selon un autre aspect, est également proposé un procédé de synthèse d'un radio-traceur dans une cassette telle que décrite précédemment comportant au moins :
- une étape d'injection dans le circuit microfluidique d'un précurseur via l'au moins une prise d'alimentation par une fiole et d'un radio-isotope via le port isotope ;
- une étape de mélange du précurseur et du radio-isotope dans au moins l'une des chambres de mélange ou des chambres de réaction ;
- une étape de synthèse du radio-traceur par réaction entre le précurseur et le radio-isotope dans au moins l'une des chambres de réaction ;
- une étape d'élution du radio-traceur par un solvant injectable à un individu dans au moins l'une des chambres de réaction ;
- une étape de dilution du radio-traceur dans une solution de NaCl dans la chambre de formulation ; et
- une étape de remplissage d'une seringue de la solution de NaCl comportant le radio-traceur via le port seringue (SP).

Par exemple, le solvant de l'étape d'élution est par exemple de l'éthanol ou une solution de dihydrogénophosphate de sodium.

Dans un exemple de mise en oeuvre, l'au moins une étape de réaction comporte une étape de réaction en température dans la chambre de réaction en température.

Dans un exemple de mise en oeuvre, le procédé comporte en outre une étape de bullage dans la chambre de formulation pour mieux mélanger un contenu de la chambre de formulation.

Une telle cassette comme le procédé associé présente par exemple au moins certains des avantages suivants :
- Limitation des déchets produits par radio-pharmaceutiques,
- Limitation des volumes morts et des pertes dans les canaux, notamment grâce aux vannes intégrées et aux canaux plus petits et plus courts (les capillaires),
- Optimisation des volumes utilisés de réactifs/billes/précurseurs, ce qui réduit les coûts correspondant,
- Temps de synthèse total diminué,
- Limite fortement le nombre d'entrées/sorties, donc limite les risques de fuites et notamment de fuites radioactives,
- Amélioration des rendements de synthèses.

L'invention, selon un exemple de réalisation, sera bien comprise et ses avantages apparaitront mieux à la lecture de la description détaillée qui suit, donnée à titre indicatif et nullement limitatif, en référence aux dessins annexés dans lesquels :
La figure 1 montre un exemple de diagramme fonctionnel d'architecture d'une cassette selon un exemple de réalisation selon l'invention,
La figure 2a présente une vue en perspective d'une cassette selon un exemple de réalisation, et la figure 2b montre un exemple de réalisation d'une chambre de mélange,
La figure 3, comportant les figures 3.1 à 3.23, illustre des étapes de procédé de synthèse d'un radio-traceur à partir d'un radio-isotope de fluor 18 (¹⁸F) selon un exemple de mise en oeuvre de l'invention,
La figure 4, comportant les figures 4.1 à 4.7, illustre des étapes de procédé de synthèse d'un radio-traceur à partir d'un radio-isotope de carbone 11 (¹¹C) selon un exemple de mise en oeuvre de l'invention, et
La figure 5, comportant les figures 5.1 à 5.17, illustre des étapes de procédé de synthèse d'un radio-traceur à partir d'un radio-isotope de gallium 68 (⁶⁸Ga) selon un exemple de mise en oeuvre de l'invention.

Les éléments identiques représentés sur les figures précitées sont identifiés par des références numériques identiques.

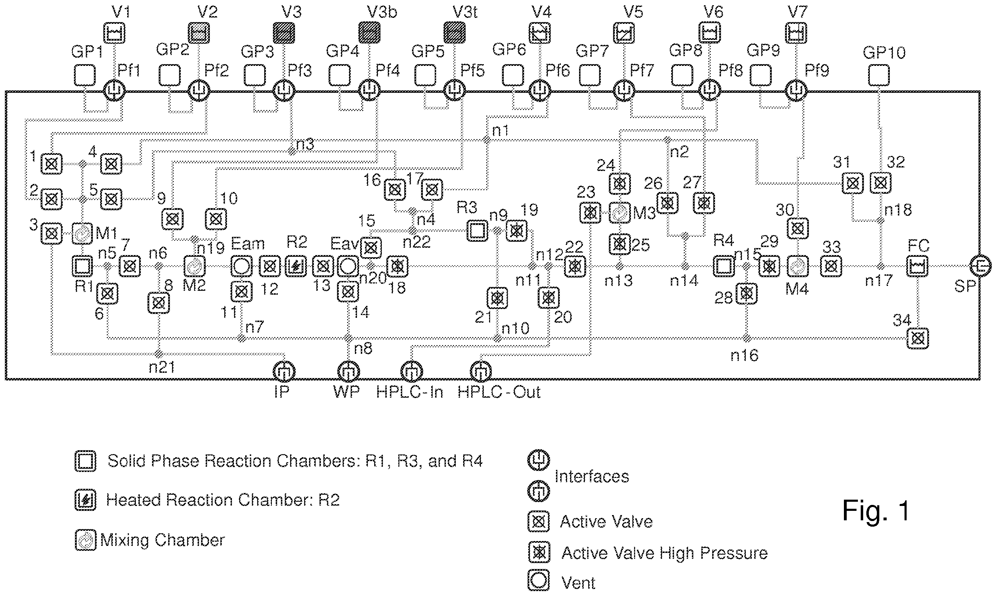

La figure 1 présente un exemple de diagramme fonctionnel d'architecture d'une cassette selon un exemple de réalisation de l'invention.

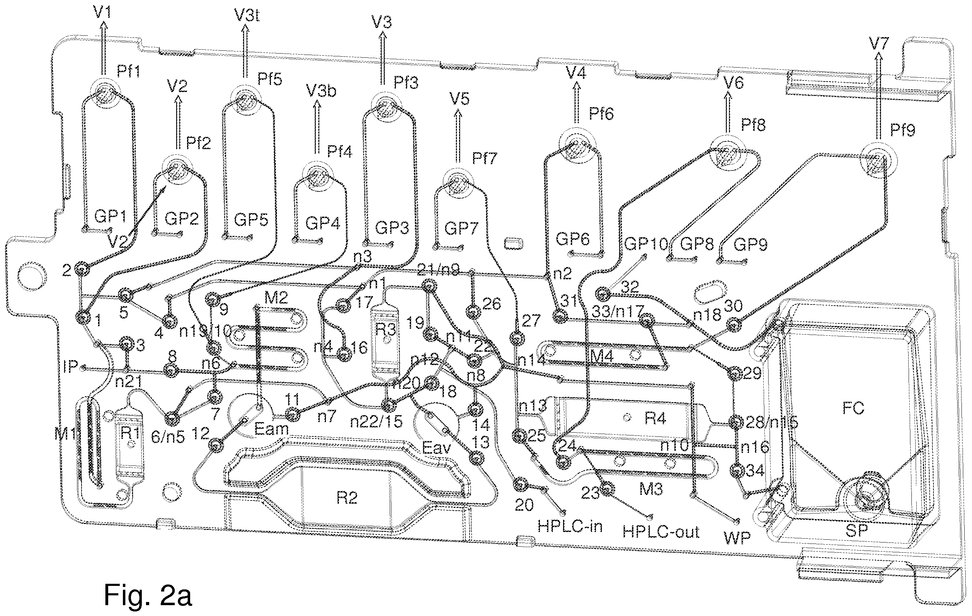

Une telle cassette comporte une carte de support, représentée figure 2a selon un exemple de réalisation de l'invention.

La carte de support mesure par exemple 190 mm de longueur, 116 mm de largeur et 47 mm de hauteur.

Une carte de support d'une cassette comporte une série d'interfaces permettant des entrées et sorties de fluides, c'est-à-dire de liquides et/ou de gaz, dans ou hors un circuit microfluidique.

Certaines de ces interfaces sont par exemple des prises d'alimentation par une fiole Pf, ici schématisées alignées en haut du diagramme.

Une prise d'alimentation par une fiole est par exemple un trou formé dans la carte de support, auquel se raccorde ensuite au moins un capillaire.

La carte de support est ici raccordée avec 9 fioles ; les fioles sont ici référencées V1, V2, V3, V3b, V3t, V4, V5, V6 et V7. Les prises d'alimentation correspondantes sont ici référencées Pf1 à Pf9.

Parmi les fioles, des fioles V1, V2, V3, V3b, V3t, V5 ont une capacité de 6 mL et des fioles V4, V6, V7 ont une capacité de 19 mL.

Une autre de ces interfaces est par exemple une prise de connexion pour une seringue SP, également dénommée port seringue SP.

Dans le cadre de la présente demande, une seringue désigne tout récipient permettant de récupérer le radio-traceur obtenu en sortie de la cassette ; cette dénomination inclut par exemple ici un réservoir ou un flacon.

Le port seringue SP est ici représenté à droite du diagramme.

Le port seringue SP comporte par exemple un connecteur. Il s'agit par exemple d'un connecteur de type Tego^{®} D-1000 de Victus du commerce.

Le port seringue SP comporte aussi par exemple un port Luer sur lequel le connecteur est monté.

Et par exemple, ce connecteur comporte une partie mâle, pour assurer une connexion étanche et normalement close, à laquelle est ensuite raccordé un filtre stérilisant, lui-même connecté à la seringue. Autrement dit, le connecteur est placé entre le port Luer et le filtre stérilisant, via lequel peut être connectée la seringue.

Une autre de ces interfaces est par exemple le port isotope IP qui est configuré pour introduire un radio-isotope dans le circuit microfluidique.

Une autre de ces interfaces est par exemple un port déchet WP. Un tel port déchet permet notamment d'extraire et/ou jeter au rebut des produits intermédiaires et/ou des surplus.

D'autres de ces interfaces sont des prises d'entrée/sortie avec une colonne HPLC. Ainsi, le port HPLC-in permet une entrée dans la colonne HPLC, donc une sortie de la cassette, et le port HPLC-out permet une sortie de la colonne HPLC, donc une entrée dans la cassette après traversée de la colonne HPLC. Pour cela des vannes rhéodynes peuvent être positionnées entre les ports HPLC-in et HPLC-out et la colonne.

Dans le présent exemple de réalisation, chaque port (HPLC in/out, isotope, déchet) n'est ici formé que d'un trou ; les parties permettant d'assurer une connexion étanche (joint ou connecteur par exemple) sont par exemple placées sur l'élément à raccorder correspondant. Bien entendu, chaque port pourrait toutefois comporter au moins un élément de raccord étanche, comme par exemple un joint.

De plus, la cassette comporte au moins un port gaz GP, et ici dix ports gaz GP. Les port gaz GP sont ici référencés GP1 à GP10.

Dans un exemple de réalisation non représenté, au moins l'un des ports gaz GP se comporte un évent hydrophobe.

Dans le présent exemple de réalisation, les ports gaz GP représentent des arrivées de gaz et sont formés d'un trou.

Les ports gaz référencés GP1 à GP9 sont associés à chacune des prises d'alimentation par une fiole Pf.

Ainsi, ces arrivées de gaz se font dans les différentes fioles, référencées de Pf1 à Pf9, lors d'une utilisation de la cassette. En injectant du gaz dans la fiole correspondante, la pression de gaz permet de chasser le fluide, ou liquide, qu'elle contient par un capillaire distinct de celui par lequel le gaz est injecté.

Dans un exemple de réalisation, la cassette peut comporter par exemple un « spike » adjoint à au moins l'une des prises d'alimentation par une fiole ; un spike est par exemple un connecteur de forme conique comportant au moins deux capillaires dont l'un communique avec la prise d'alimentation par une fiole et l'autre avec le port gaz correspondant et sur lequel est connectée une fiole lors d'une utilisation de la cassette.

Le port gaz GP10 est relié directement à une chambre de formulation FC, décrite ci-après, par un capillaire et le port gaz GP10 est configuré pour alimenter en gaz cette chambre de formulation FC, notamment lors du remplissage d'une seringue, via le port seringue SP, pour limiter, ou même éviter, une dépression.

Dans le présent exemple de réalisation, le circuit microfluidique comporte quatre chambres de réaction R1, R2, R3, R4.

Ici, les chambres de réaction ont les capacités suivantes : R1 et R3 : 50 µL, R2 : 300 µL et R4 : 200 µL.

Les chambres de réaction R1, R3 et R4 sont des chambres de réaction classiques.

En outre, elles sont ici chargées en billes.

Par exemple la chambre de réaction R1 comporte des billes QMA lorsqu'elle est utilisée avec du fluor 18 ou du gallium 68. Pour une synthèse à base de carbone 11 elle peut être vide par exemple.

Les billes ont ici un diamètre d'au moins 30 µm.

La chambre de réaction R2 est une chambre de réaction en température.

Ainsi, lorsque la cassette est positionnée dans une installation, la chambre de réaction en température R2 est reliée à un élément chauffant/refroidissant par exemple.

La chambre de réaction en température R2 est de préférence physiquement séparée du reste de la cassette, par exemple par une découpe dans la carte comme l'illustre la figure 2a, permettant ainsi de ne pas chauffer les structures autour de la chambre de réaction en température R2 par conduction thermique.

La chambre de réaction en température R2 permet par exemple de faire réagir un précurseur, contenu dans une des fioles, par exemple la fiole V3b ou la fiole V5, avec l'élément radioactif introduit par le port isotope IP à des températures différentes de la température ambiante si nécessaire.

Le circuit microfluidique comporte aussi des chambres de mélange M1, M2, M3, M4.

Les chambres de mélange M1 à M4 sont par exemple des capillaires avec des microstructures qui créent des vortex dans les écoulements fluides et ainsi augmentent fortement le mélange entre deux fluides.

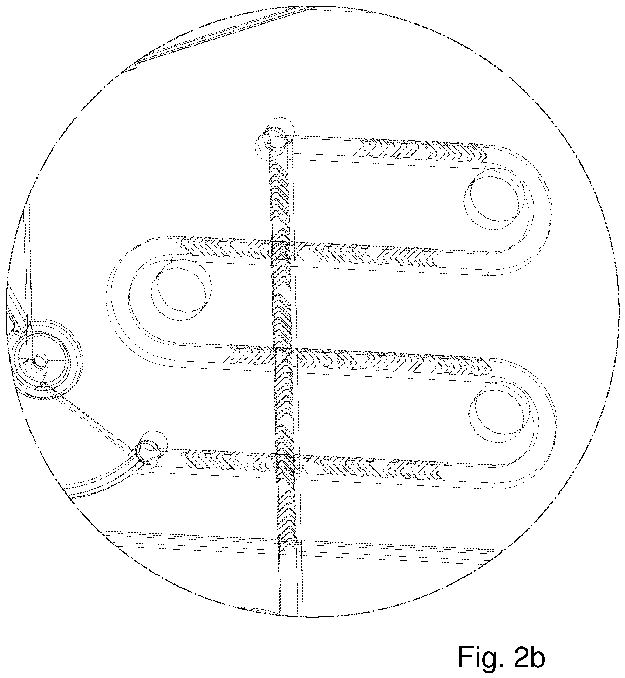

Une telle chambre de mélange est par exemple illustrée en figure 2b qui est un agrandissement de la chambre de mélange M2.

Devant, c'est-à-dire en amont des chambres de réaction R1, R2 et R4, se trouvent les chambres de mélange M1, M2 et M3. La chambre de mélange M4 est en aval de la chambre de réaction R4 et en amont de la chambre de formulation FC.

La chambre de mélange M2, positionnée en amont de la chambre de réaction en température R2, permet notamment de mélanger le précurseur au radio-isotope.

La chambre de mélange M3, positionnée en amont de la chambre de réaction R4 permet par exemple de diminuer une concentration de CH₃CN en sortie d'HPLC avant un changement de solvant dans la chambre de réaction R4 et ainsi favoriser un changement de solvant performant.

Enfin, le circuit microfluidique comporte la chambre de formulation FC.

La chambre de formulation a par exemple une capacité de l'ordre du millilitre, par exemple de la dizaine de millilitres, par exemple d'environ 12 mL dans le présent exemple de réalisation.

Elle est possiblement dotée d'un port en sa partie supérieure pour permettre une évacuation de gaz lors de son remplissage par exemple.

La carte schématisée figure 1 comporte trente-quatre vannes permettant le pilotage des fluides en provenance des différentes interfaces. Les vannes sont numérotées de 1 à 34. Ces vannes par exemple sont pilotées unitairement par énergie pneumatique par l'intermédiaire du contrôle commande. Elles sont par exemple actionnées à des pressions de 0-7 bars mais de préférence autour de 3-4 bars.

Ainsi, la carte de support ne contient aucun réactif embarqué, hormis les billes.

Les réactifs sont tous apportés par les fioles numérotées de V1 à V7 et le radio-isotope vient par le port isotope.

L'utilisation et l'emplacement des différentes fioles est ainsi adaptable en fonction de la synthèse que le radiochimiste souhaite réaliser.

Ainsi, dans le présent exemple d'architecture, la chambre de mélange M1 est reliée par un capillaire à la fiole V1 (capillaire qui comporte la vanne 2), par un capillaire à la fiole V2 (capillaire qui comporte la vanne 1), par un capillaire à la fiole V3 (capillaire qui comporte la vanne 5) et par un capillaire à la fiole V4 (capillaire qui comporte la vanne 4).

De plus, la vanne 1, la vanne 2, la vanne 4 et la vanne 5 sont reliées entre elles par des capillaires.

Le capillaire entre la vanne 4 et la fiole V4 comporte un noeud n1 auquel se raccorde un capillaire, comportant la vanne 17, qui se raccorde à la chambre de réaction R3, de sorte que la chambre de mélange M1 est en outre reliée à la chambre de réaction R3 par un capillaire (qui comporte la vanne 17).

La chambre de mélange M1 est en outre reliée à la chambre de réaction R4 par un capillaire (qui comporte en outre la vanne 26) et à la chambre de formulation FC par un capillaire (qui comporte en outre la vanne 31).

Un noeud n2 raccorde le capillaire entre le noeud n1 et la vanne 31 au capillaire comportant la vanne 26.

Le capillaire entre la vanne 5 et la fiole V3 comporte un noeud n3 auquel se raccorde un capillaire, comportant la vanne 16, qui se raccorde à la chambre de réaction R3, de sorte que la chambre de mélange M1 est en outre reliée à la chambre de réaction R3 par un capillaire (qui comporte la vanne 16).

Un noeud n4 sur le capillaire entre la vanne 16 et la chambre de réaction R3 raccorde un capillaire à la vanne 17, et un noeud n22 entre la chambre de réaction R3 et le noeud n4 permet également un raccord par un capillaire à la vanne 15.

La chambre de mélange M1 est aussi raccordée au port isotope IP par un capillaire qui comporte la vanne 3.

Enfin, la chambre de mélange M1 est raccordée à la chambre de réaction R1, en amont de celle-ci par un capillaire.

La chambre de réaction R1 est donc d'une part raccordée à la chambre de mélange M1 et d'autre part à la chambre de mélange M2, au port isotope, au port déchet et à la chambre de réaction en température par des capillaires.

Le capillaire entre la chambre de réaction R1 et la chambre de mélange M2 comporte la vanne 7.

Entre la chambre de réaction R1 et la vanne 7 un noeud n5 raccorde un capillaire comportant la vanne 6 et menant au port déchet WP.

Entre la vanne 7 et la chambre de mélange M2 le capillaire comporte un noeud n6 auquel se raccorde un capillaire comportant la vanne 8 et qui se raccorde au capillaire entre la vanne 3 et le port isotope IP en un noeud n21 pour relier la chambre de réaction R1 au port isotope IP.

La chambre de mélange M2 est raccordée à la fiole V3b par un capillaire qui comporte la vanne 9 et à la fiole V3t par un capillaire qui comporte la vanne 10 qui se raccorde au capillaire entre la chambre de mélange M2 et la vanne 9 en un noeud n19.

Entre la chambre de mélange M2 et la chambre de réaction en température R2, le capillaire comporte un évent Eam et la vanne 12 (laquelle est entre l'évent et la chambre de réaction en température R2) qui sont donc considérés en amont de la chambre de réaction en température R2.

Ledit évent amont Eam est relié par un capillaire comportant la vanne 11 au capillaire entre la vanne 6 et le port déchet WP en un noeud n7.

Entre la vanne 15 et la chambre de réaction en température R2, le capillaire comporte un évent Eav et la vanne 13 (laquelle est entre l'évent et la chambre de réaction en température R2) qui sont donc considérés en aval de la chambre de réaction en température R2.

Ledit évent aval Eav est relié par un capillaire comportant la vanne 14 au capillaire entre le noeud n7 et le port déchet WP, en un noeud n8.

La chambre de réaction R3 est donc reliée d'une part aux vannes 15, 16 et 17 ; et d'autre part au port déchet WP, au port HPLC-in, à la chambre de formulation FC, à la chambre de mélange M3 et à la chambre de réaction R4.

La chambre de réaction R3 est reliée au port déchet WP par un capillaire comportant la vanne 21 raccordé en un noeud n10 au capillaire entre le noeud n8 et la chambre de formulation FC.

Entre la chambre de réaction R3 et la vanne 21 le capillaire comporte un noeud n9 auquel se raccorde un capillaire, comportant la vanne 19 et la vanne 22, qui se raccorde à la chambre de réaction R4.

Entre les vannes 19 et 22, un noeud n11 raccorde un capillaire comportant la vanne 18 au capillaire entre la vanne 15 et l'évent aval en un noeud n20.

Entre le noeud 11 et la vanne 22, un noeud n12 raccorde un capillaire comportant la vanne 20 au port HPLC-in.

La chambre de mélange M3 est reliée à la fiole V6 par un capillaire qui comporte la vanne 24, au port HPLC-out par un capillaire qui comporte la vanne 23, et au capillaire entre la vanne 22 et la chambre de réaction R4 en un noeud n13 par un capillaire qui ici comporte la vanne 25. Ainsi, la chambre de mélange M3 est aussi reliée au moins au port HPLC-in, voire aussi à la chambre de réaction en température R2, la chambre de réaction R3 et aux fioles V3 et V4 par exemple.

La chambre de réaction R4 est raccordée à la fiole V5 par un capillaire comportant la vanne 27.

Un noeud n14 permet de raccorder les capillaires entre la vanne 26, la vanne 27, au capillaire entre le noeud n13 et la chambre de réaction R4.

La chambre de réaction R4 est aussi raccordée à la chambre de mélange M4 par un capillaire comportant la vanne 29.

Entre la chambre de réaction R4 et la vanne 29, un noeud n15 permet de raccorder un capillaire comportant la vanne 28 à un capillaire entre le noeud n8 et la vanne 34 en un noeud n16.

La chambre de mélange M4 est considérée en aval de la chambre de réaction R4, reliée à celle-ci par un capillaire comportant la vanne 29, et en amont de la chambre de formulation FC à laquelle elle est reliée par un capillaire comportant la vanne 33.

La chambre de mélange M4 est aussi parallèlement reliée à la chambre de formulation FC par des capillaires comportant les vannes 29, 28 et 34.

Autrement dit, le capillaire entre la chambre de formulation FC et le noeud n16 comporte la vanne 34.

La chambre de mélange M4 est aussi reliée à la fiole V7 par un capillaire comportant la vanne 30.

Entre la vanne 33 et la chambre de formulation FC, le capillaire comporte un noeud n17 raccordant un capillaire comportant la vanne 32 au port gaz GP10.

Entre la vanne 32 et le noeud n17, le capillaire comporte un noeud n18 auquel se raccorde le capillaire comportant la vanne 31.

La vanne 31 est donc entre le noeud n2 et le noeud n18.

Enfin, la chambre de formulation est au moins raccordée à la chambre de mélange M4 par le capillaire comportant la vanne 33, au port gaz GP10 par le capillaire comportant la vanne 32, à la fiole V4 par le capillaire comportant la vanne 31 et au port déchet WP par le capillaire comportant la vanne 34. Elle est aussi raccordée au port seringue SP par un capillaire.

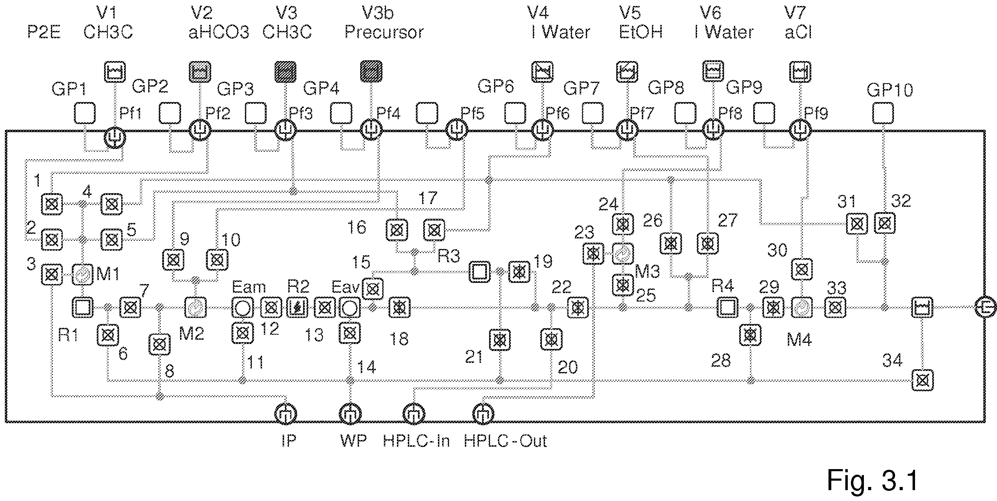

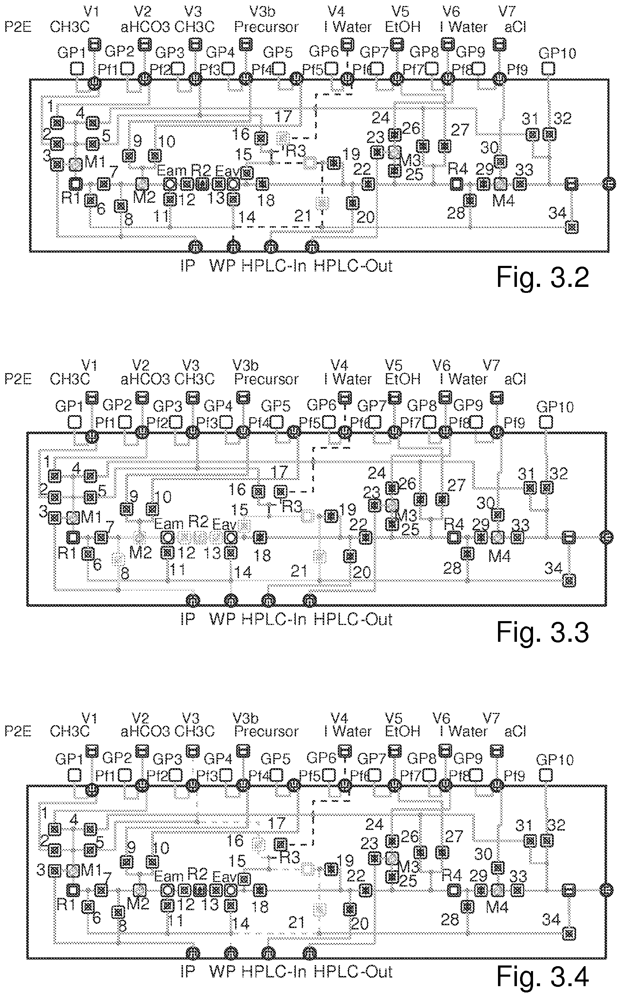

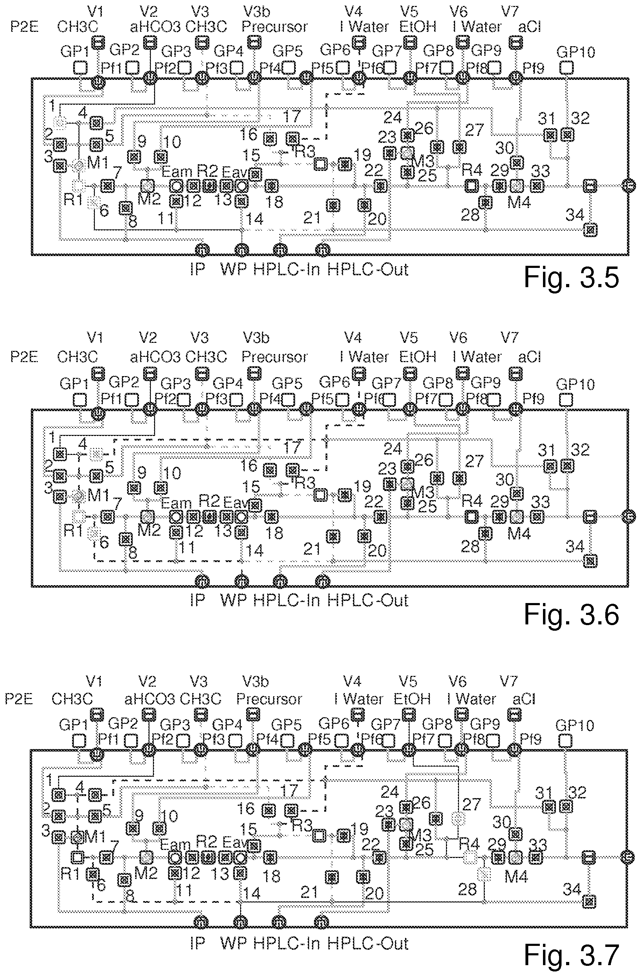

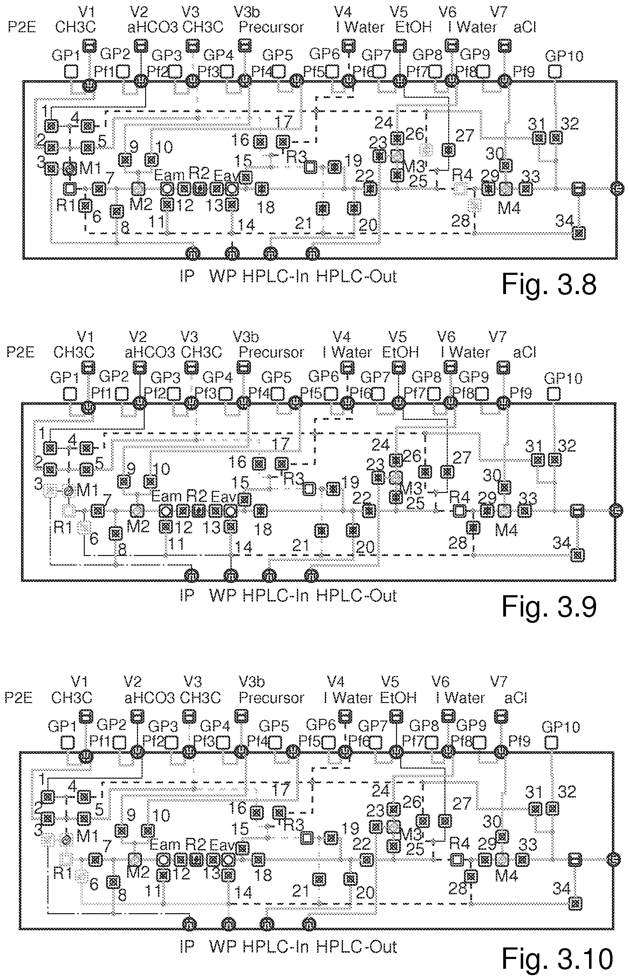

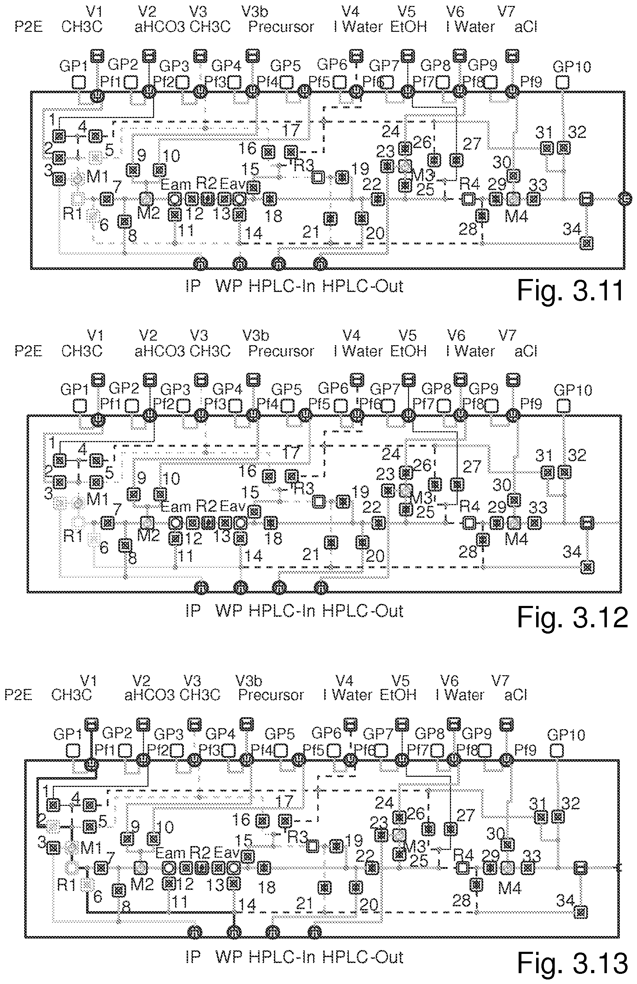

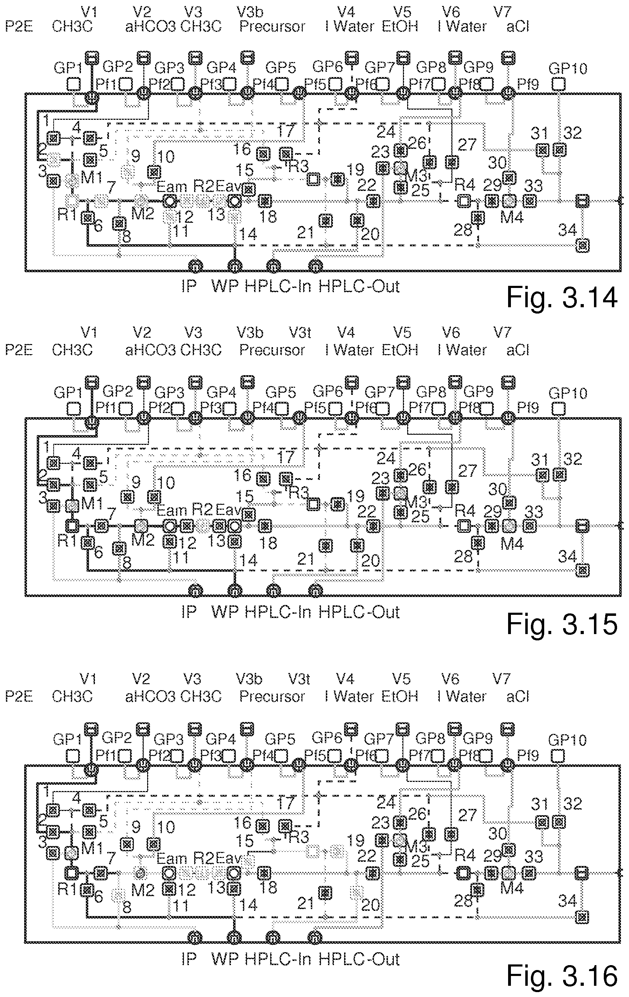

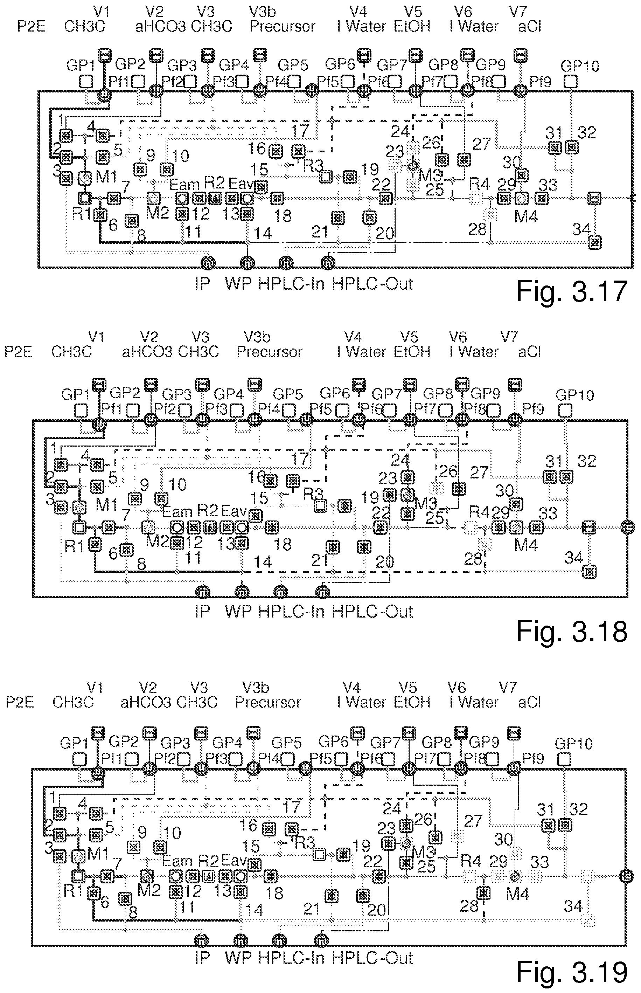

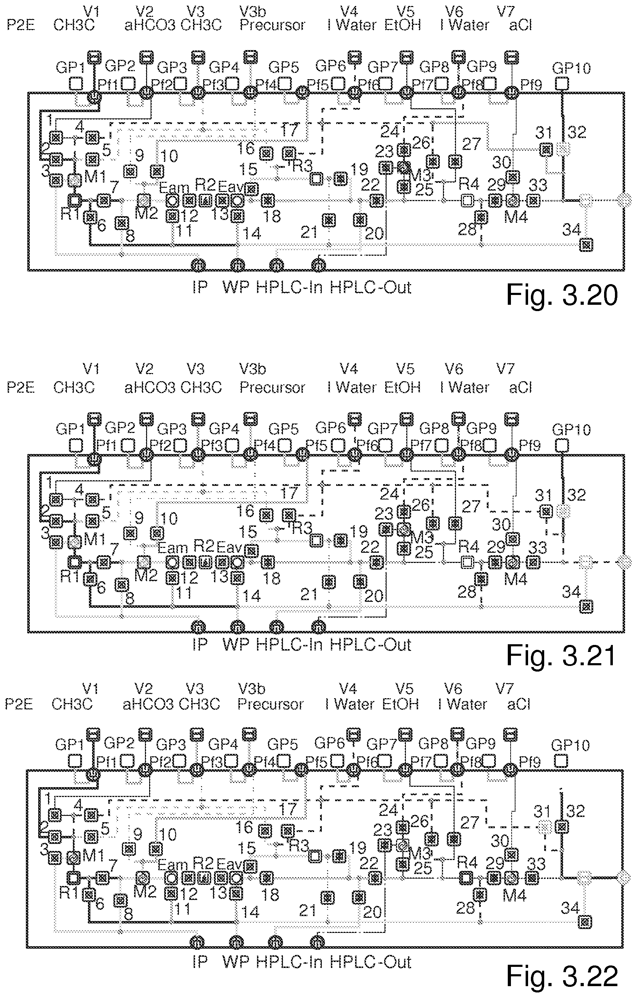

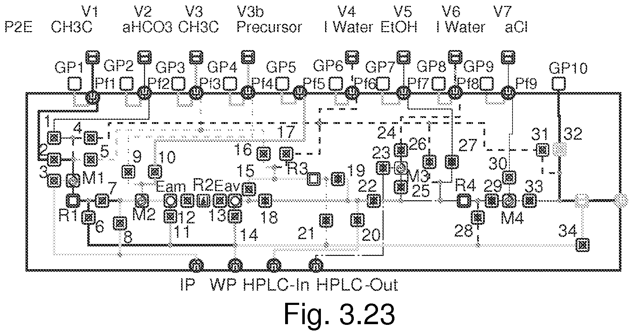

Les figures 3, 4 et 5 permettent d'illustrer une utilisation d'une telle cassette dans un procédé de synthèse de radio-traceurs à partir de radio-isotopes différents, en l'occurrence à partir de fluor 18, de carbone 11 et de gallium 68.

La figure 3.1 illustre la configuration de la cassette pour une utilisation avec du fluor 18, pour une substitution nucléophile aliphatique.

Huit fioles sont utilisées :
- V1 remplie avec 4 mL de P2ET dilué dans du CH₃CN (aussi noté P2ET>CH₃CN)
- V2 remplie avec 4 mL de NaHCOs
- V3 remplie avec 4 mL de CH₃CN
- V3b remplie avec 4 mL de précurseur
- V4 remplie avec 15mL d'eau déminéralisée (« DI water »)
- V5 remplie avec 4 mL de EtOH
- V6 remplie avec 15 mL d'eau déminéralisée (« DI water »)
- V7 remplie avec 10 mL de NaCl

Les chambres utilisées sont :
- R1 remplie de billes QMA
- R2 ne contenant aucune bille
- R3 remplie de billes Al₂O₃ (alumine)
- R4 remplie de billes C18 (silicium carbone)

Les étapes de synthèse sont ensuite les suivantes :
Etape 1 (figure 3.2) : remplissage de la chambre de réaction R3 avec de l'eau déminéralisée de la fiole V4, et vidange par le port déchet WP ; ouverture des vannes 17 et 21 ;
Etape 2 (figure 3.3) : séchage de la chambre de réaction R3 par du gaz via le port isotope IP, via la chambre de mélange M2 et la chambre de réaction en température R2 ; ouverture des vannes 8, 12, 13, 15 et 21 ; fermeture de la vanne 17 ;
Etape 3 (figure 3.4) : remplissage de la chambre de réaction R3 avec du CH₃CN contenu dans la fiole V3 ; ouverture des vannes 16 et 21 ; fermeture d'au moins la vanne 15 ;
Etape 4 (figure 3.5) : remplissage de la chambre de réaction R1, via la chambre de mélange M1, avec du NaHCOs contenu dans la fiole V2, et vidange par le port déchet WP ; ouverture des vannes 1 et 6 ; fermeture des vannes 16 et 21 ;
Etape 5 (figure 3.6) : remplissage de la chambre de réaction R1, via la chambre de mélange M1, avec de l'eau déminéralisée contenue dans la fiole V4, et vidange par le port déchet WP ; ouverture des vannes 4 et 6 ; fermeture de la vanne 1 ;
Etape 6 (figure 3.7) : remplissage de la chambre de réaction R4 avec EtOH de la fiole V5, et vidange par le port déchet WP ; ouverture des vannes 27 et 28 ; fermeture des vannes 4 et 6 ;
Etape 7 (figure 3.8) : remplissage de la chambre de réaction R4 avec de l'eau déminéralisée de la fiole V4, et vidange par le port déchet WP ; ouverture des vannes 26 et 28 ; fermeture de la vanne 27 ;
Etape 8 : étape menée dans la colonne HPLC, hors de la carte ;
Etape 9 (figure 3.9) : injection de l'isotope ¹⁸F sous forme anionique en solution (dans de l'eau enrichie) par le port isotope IP dans la chambre de réaction R1, via la chambre de mélange M1, piégeage d'au moins une partie des ions fluor par les billes de la chambre de réaction R1 et évacuation d'au moins une partie de l'eau enrichie par le port déchet WP ; ouverture des vannes 3 et 6 ; fermeture des vannes 26 et 28 ;
Etape 10 (figure 3.10) : séchage de la chambre de réaction R1, via la chambre de mélange M1, par du gaz via le port isotope IP ; ouverture, ou maintien en ouverture, des vannes 3 et 6 ;
Etape 11 (figure 3.11) : rinçage de la chambre de réaction R1, via la chambre de mélange M1, avec du CH₃CN de la fiole V3 et évacuation par le port déchet WP ; ouverture des vannes 5 et 6 ; fermeture de la vanne 3 ;
Etape 12 (figure 3.12) : séchage de la chambre de réaction R1, via la chambre de mélange M1, par du gaz vecteur via le port isotope IP ; ouverture des vannes 3 et 6 ; fermeture de la vanne 5 ;
Etape 13 (figure 3.13) : injection d'environ 50 µL de P2ET>CH3CN de la fiole V1 dans la chambre de réaction R1, via la chambre de mélange M1, et évacuation par le port déchet WP ; ouverture des vannes 2 et 6 ; fermeture de la vanne 3 ;
Etape 14 (figure 3.14) : mélange de P2ET>CH3CN de la fiole V1 avec du précurseur de la fiole V3b dans la chambre de mélange M2, puis réaction dans la chambre de réaction en température R2 ; ouverture des vannes 2, 7, 9, 12 et 13, et des vannes 11 et 14 ; fermeture de la vanne 6 ; évacuation de bulles via des évents en amont et aval de la chambre de réaction en température R2 si nécessaire ;
Etapes 15-17 (figure 3.15) : chauffage de la chambre de réaction en température R2, établissement de la réaction et refroidissement ; fermeture d'au moins les vannes 2, 7, 9, 11, 12, 13 et 14 ;
Etape 18 (figure 3.16) : poussée vers la colonne HPLC par le port HPLC-in, via la chambre de mélange M2 et la chambre de réaction R3, du contenu de la chambre de réaction en température R2 par du gaz introduit par le port isotope IP ; ouverture des vannes 8, 12, 13, 15, 19 et 20 ;
Etape 19-23 : étapes menées dans la colonne HPLC ;
Etape 24 (figure 3.17) : après traversée de la colonne HPLC, introduction du radio-traceur purifié par le port HPLC-out, mélange avec de l'eau déminéralisée de la fiole V6 dans la chambre de mélange M3 et injection dans la chambre de réaction R4 puis le liquide est évacué par le port déchet WP ; ouverture des vannes 23, 24, 25 et 28 ; fermeture des vannes 8, 12, 13, 15, 19 et 20 ;
Etape 25 (figure 3.18) : nettoyage de la chambre de réaction R4 avec de l'eau déminéralisée de la fiole V4 et évacuation par le port déchet WP ; le radio-traceur purifié étant fixé aux billes de la chambre de réaction R4 ; ouverture des vannes 26 et 28 ; fermeture des vannes 23, 24 et 25 ;
Etapes 26-27 (figure 3.19) : élution et changement de solvant par EtOH de la fiole V5, mélange avec NaCl de la fiole V7 dans la chambre de mélange M4, positionnée en aval de la chambre de réaction R4, et injection dans la chambre de formulation FC, évacuation d'une partie par le port déchet WP ; ouverture des vannes 27, 29, 30, 33 et 34 ; fermeture des vannes 26 et 28 ;
Etape 28-29 (figure 3.20) : remplissage d'une seringue via le port seringue SP par du contenu de la chambre de formulation FC, à cet effet injection de gaz par le port gaz GP10 ; ouverture de la vanne 32 ; fermeture des vanne 27, 29, 30, 33 et 34 ; un filtre est présent hors de la carte ; puis remplissage d'une seringue pour un contrôle qualité de la même manière ;
Etape 30 (figure 3.21) : séchage du filtre positionné hors de la carte par du gaz introduit par le port GP10 ;
Etape 31 (figure 3.22) : mouillage du filtre par de l'eau déminéralisée de la fiole V4 ; ouverture de la vanne 31 et fermeture de la vanne 32 ; et
Etape 32 (figure 3.23) : réalisation d'un test à point de bulles pour vérifier l'intégrité du filtre stérilisant ; ouverture de la vanne 32 et fermeture de la vanne 31.

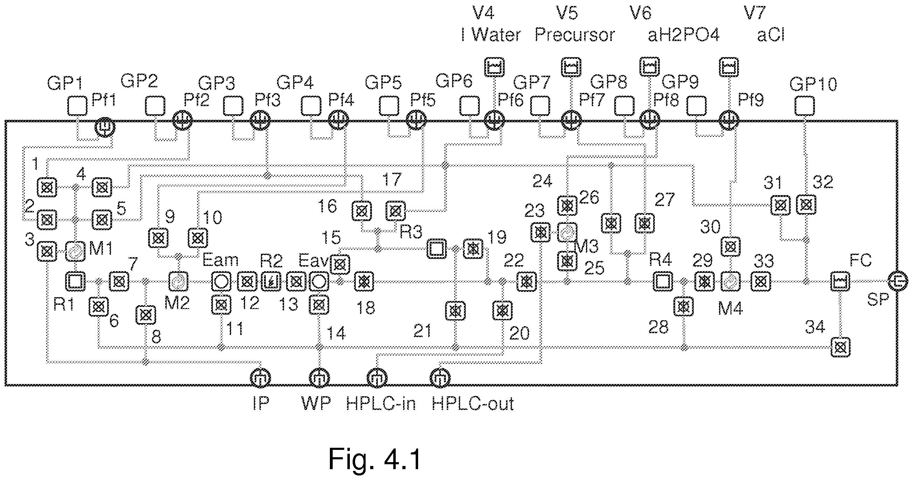

La figure 4.1 illustre la configuration de la cassette pour une utilisation avec du carbone 11 (pour une synthèse de 11C-méthionine).

Quatre fioles sont utilisées :
- V4 remplie avec 15 mL d'eau déminéralisée (« DI water »)
- V5 remplie avec 4 mL de précurseur
- V6 remplie avec 15 ml de NaH₂PO₄
- V7 remplie avec 10 ml de NaCl

Les chambres utilisées sont :
- R4 remplie de billes C18+ (silicium carbone)

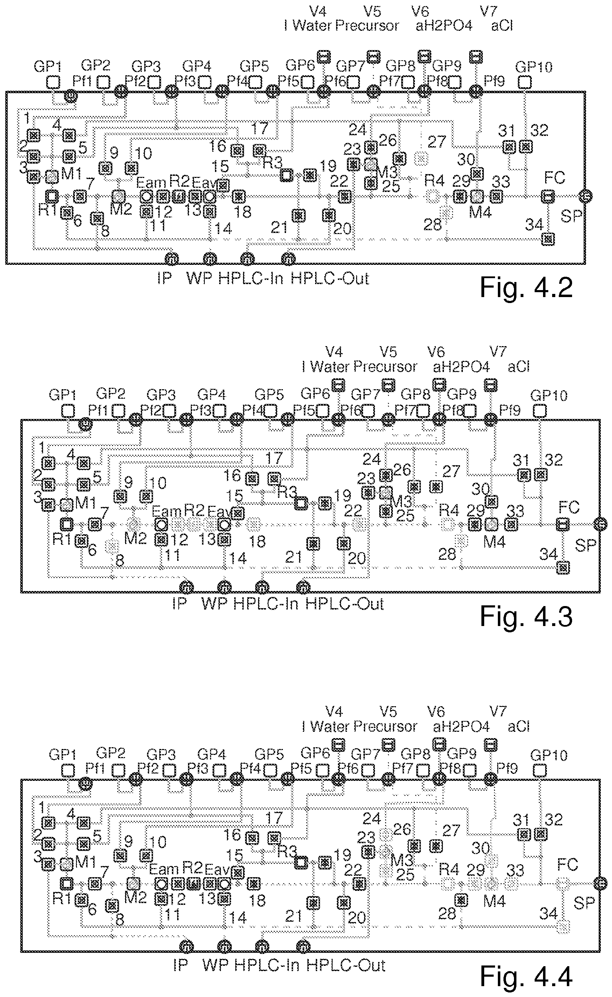

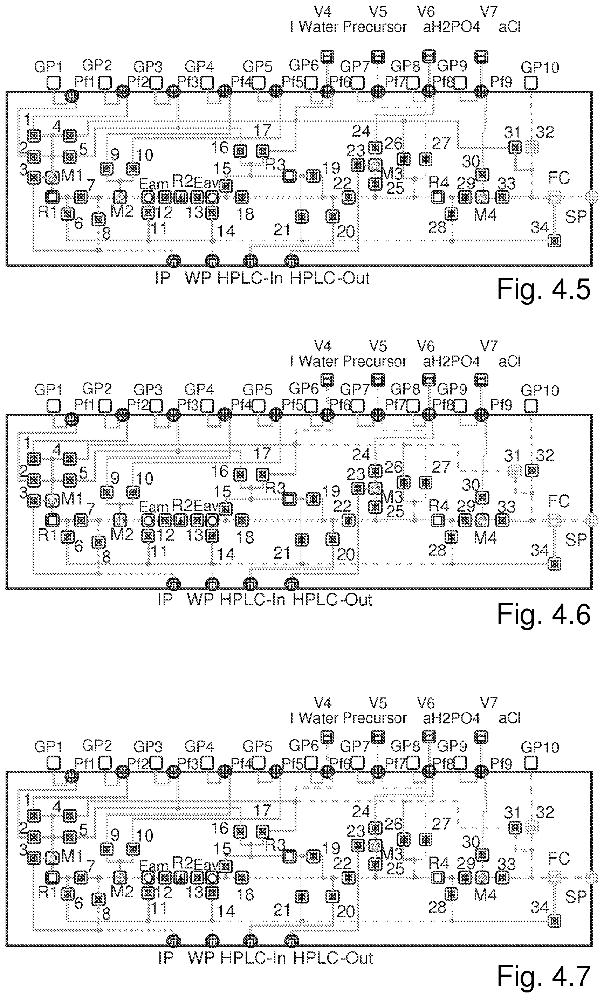

Les étapes de synthèse sont ensuite les suivantes :
Etape 1 (figure 4.2) : remplissage de la chambre de réaction R4 avec du précurseur de la fiole V5 et évacuation par le port déchet WP ; ouverture des vannes 27 et 28 ;
Etape 2 (figure 4.3) : bullage par du 11-CH₃I injecté par le port isotope IP de la chambre de réaction R4, via la chambre de mélange M2 et la chambre de réaction en température R2 et évacuation par le port déchet WP ; ouverture des vannes 8, 12, 13, 18, 22 et 28 ; fermeture de la vanne 27 ;
Etapes 3 et 4 (figure 4.4) : élution avec NaH2PO4 de la fiole V6 via la chambre de mélange M3 et la chambre de réaction R4 et mélange avec NaCl de la fiole V7 via la chambre de mélange M4 dans la chambre de formulation FC ; ouverture des vannes 24, 25, 29, 30, 33, 34 ; fermeture des vannes 8, 12, 13, 18, 22 et 28 ;
Etape 5-7 (figure 4.5) : remplissage d'une seringue pour un patient (le filtre est hors de la carte) via le port seringue SP par du contenu de la chambre de formulation FC, à cet effet injection de gaz par le port gaz GP10 ; ouverture de la vanne 32 ; fermeture des vannes 24, 25, 29, 30, 33, 34 ; puis remplissage d'une seringue pour un contrôle qualité de la même manière ; puis séchage du filtre positionné hors de la carte par du gaz introduit par le port GP10 ;
Etape 8 (figure 4.6) : mouillage du filtre par de l'eau déminéralisée de la fiole V4 ; ouverture de la vanne 31 et fermeture de la vanne 32 ; et
Etape 9 (figure 4.7) : réalisation d'un test à point de bulles ; ouverture de la vanne 32 et fermeture de la vanne 31.

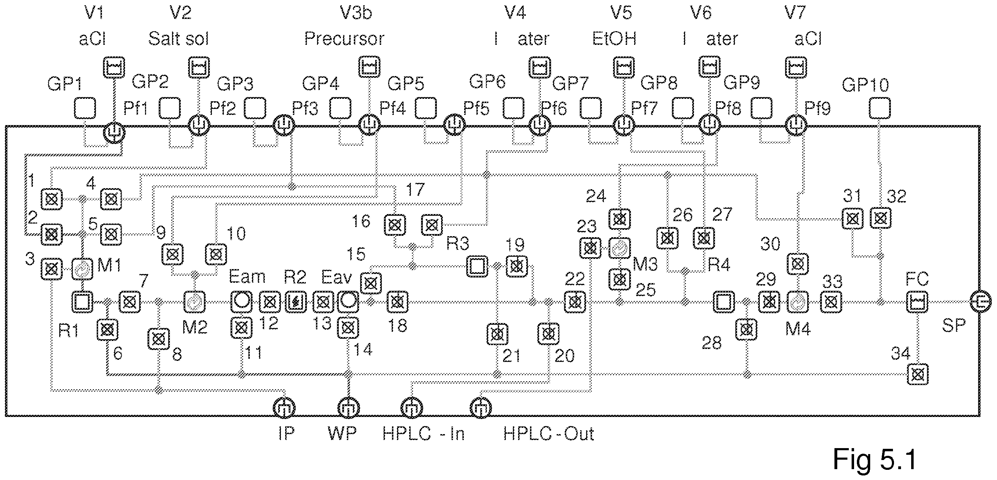

La figure 5.1 illustre la configuration de la cassette pour une utilisation avec du gallium 68 (68Ga-DOTANOC).

Huit fioles sont utilisées :
- V1 remplie avec 4 mL de NaCl
- V2 remplie avec 4 mL de solution saline
- V3b remplie avec 4 ml de précurseur
- V4 remplie avec 15 mL d'eau déminéralisée (« DI water »)
- V5 remplie avec 4 mL de EtOH
- V6 remplie avec 15 mL d'eau déminéralisée (« DI water »)
- V7 remplie avec 10 mL de NaCl

Les chambres utilisées sont :
- R1 remplie de billes QMA
- R2 ne contenant aucune bille
- R4 remplie de billes C18

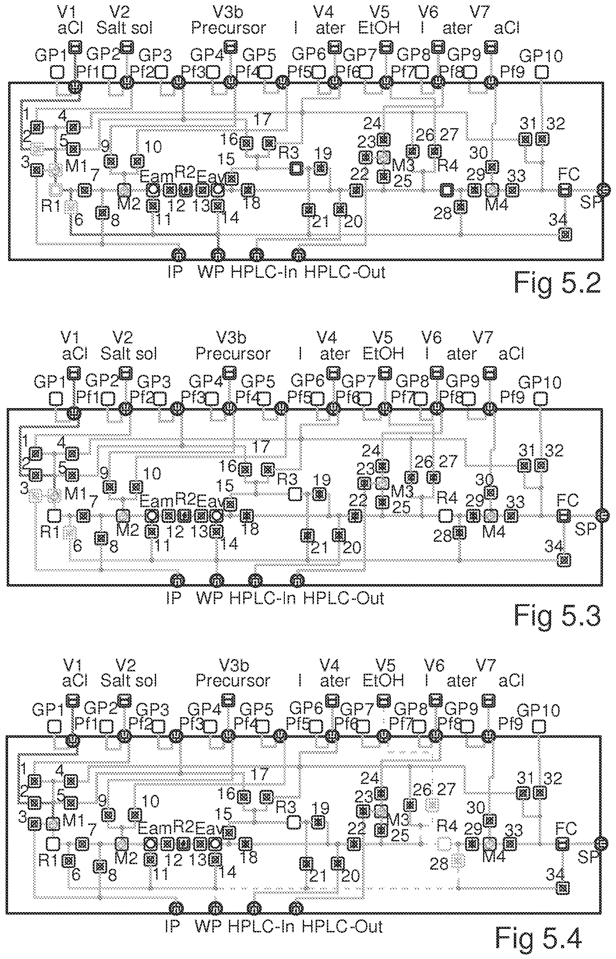

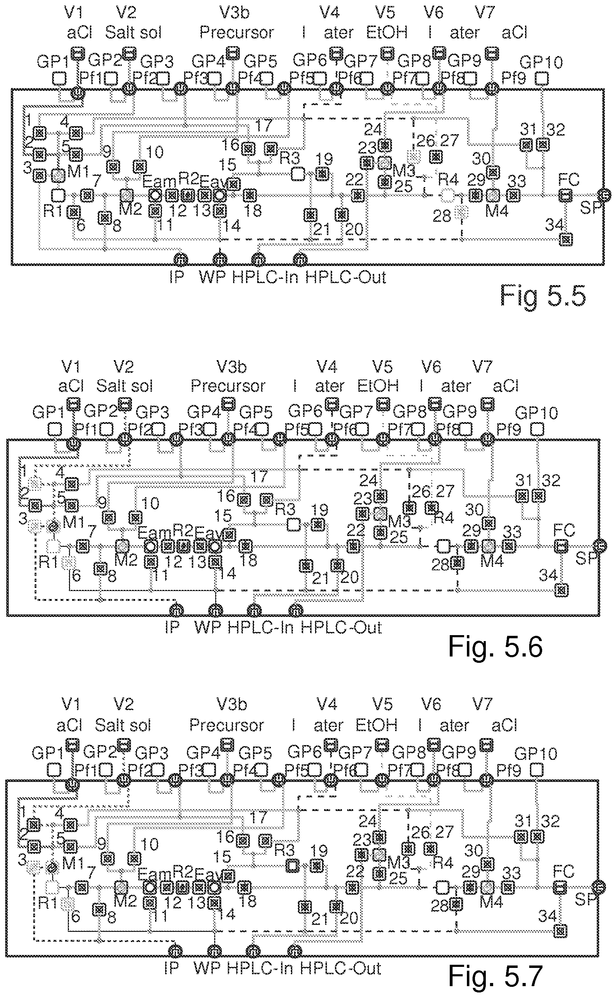

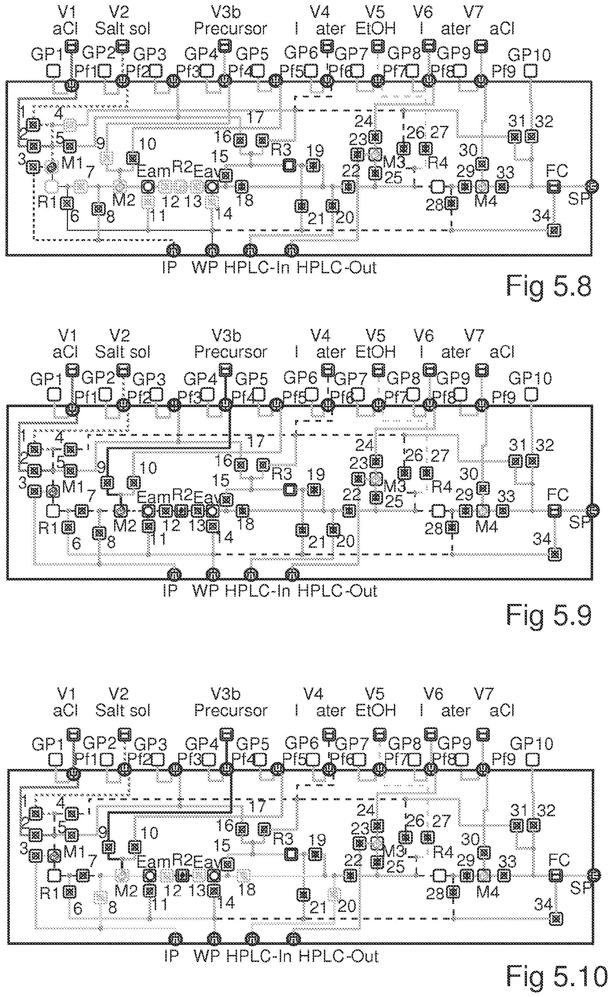

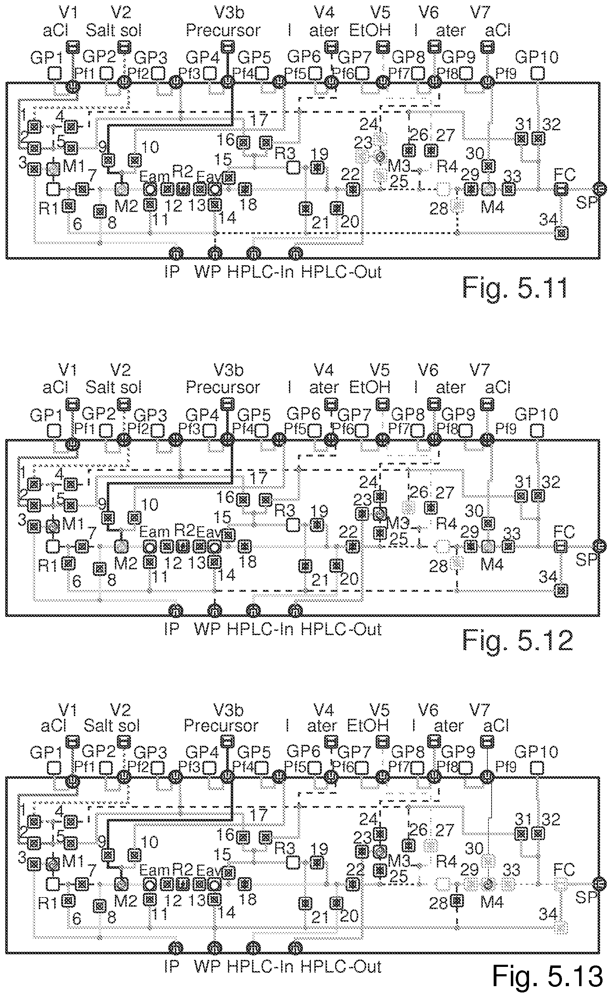

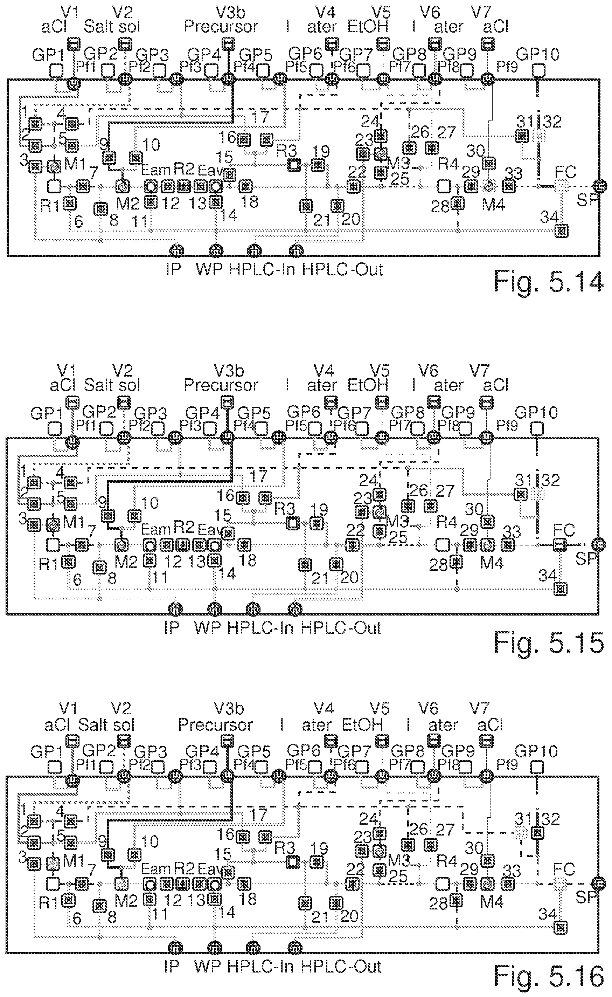

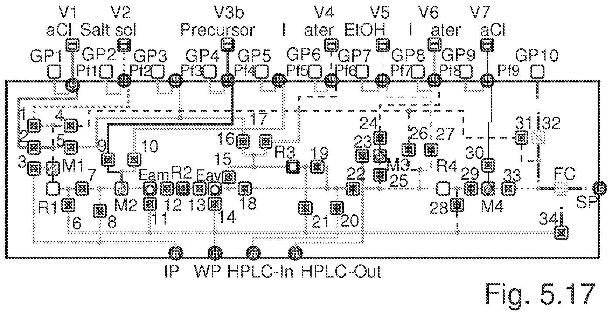

Les étapes de synthèse sont ensuite les suivantes :
Etape 1 (figure 5.2) : remplissage de la chambre de réaction R1, via la chambre de mélange M1, avec du NaCl 5M (à une concentration de 5 mol/L) de la fiole V1, et vidange par le port déchet WP ; ouverture des vannes 2 et 6 ;
Etape 2 (figure 5.3) : séchage de la chambre de réaction R1, via la chambre de mélange M1, par du gaz via le port isotope IP et évacuation par le port déchet WP ; ouverture des vannes 3 et 6 ; fermeture de la vanne 2 ;
Etape 3 (figure 5.4) : remplissage de la chambre de réaction R4 avec du EtOH contenu dans la fiole V5 et évacuation par le port déchet ; ouverture des vannes 27 et 28 ; fermeture des vannes 3 et 6 ;
Etape 4 (figure 5.5) : remplissage de la chambre de réaction R4 avec de l'eau déminéralisée contenue dans la fiole V4, et vidange par le port déchet WP ; ouverture des vannes 26 et 28 ; fermeture de la vanne 27 ;
Etape 5 : dans la colonne HPLC ;
Etape 6 (figure 5.6) : dilution du ⁶⁸Ga introduit par le port isotope IP dans la solution saline de la fiole V2 dans la chambre de mélange M1 et injection dans la chambre de réaction R1 puis évacuation d'au moins une partie de l'eau enrichie par le port déchet WP, au moins une partie du 68Ga restant fixé sur les billes de la chambre de réaction R1 ; ouverture des vannes 1, 3 et 6 ; fermeture des vannes 26 et 28 ;
Etape 7 (figure 5.7) : séchage de la chambre de réaction R1, via la chambre de mélange M1, par du gaz vecteur via le port isotope IP ; ouverture, ou maintien en ouverture, des vannes 3 et 6 ; fermeture de la vanne 1
Etape 8-9 (figure 5.8) : élution avec de l'eau déminéralisée de la fiole V4 dans la chambre de réaction R1, via la chambre de mélange M1, avec du précurseur de la fiole V3b dans la chambre de mélange M2, puis réaction dans la chambre de réaction en température R2 ; ouverture des vannes 4, 7, 9, 12 et 13, et des vannes 11 et 14 ; fermeture des vannes 3 et 6 ; évacuation de bulles via des évents en amont et aval de la chambre de réaction en température R2 si nécessaire ;
Etapes 10-12 (figure 5.9) : chauffage de la chambre de réaction en température R2, établissement de la réaction et refroidissement de la chambre de réaction en température R2 ; fermeture des vannes 4, 7, 9, 11, 12, 13 et 14 ;
Etape 13 (figure 5.10) : transfert du contenu de la chambre de réaction en température R2 vers la colonne HPLC par le port HPLC-in, par une injection de gaz par le port isotope IP, via la chambre de mélange M2 ; ouverture des vannes 8, 12, 13, 18 et 20 ;
Etape 14-17 : dans la colonne HPLC ;
Etape 18 (figure 5.11) : après traversée de la colonne HPLC, introduction du radio-traceur purifié par le port HPLC-out, mélange avec de l'eau déminéralisée de la fiole V6 dans la chambre de mélange M3 et injection dans la chambre de réaction R4 puis le liquide est évacué par le port déchet WP ; ouverture des vannes 23, 24, 25 et 28 ; fermeture des vannes 8, 12, 13, 18 et 20 ;
Etape 19 (figure 5.12) : nettoyage de la chambre de réaction R4 avec de l'eau déminéralisée de la fiole V4 et évacuation par le port déchet WP, le radio-traceur étant fixé sur les billes de la chambre de réaction R4 ; ouverture des vannes 26 et 28 ; fermeture des vannes 23, 24 et 25 ;
Etapes 20-21 (figure 5.13) : élution du radio-traceur par EtOH de la fiole V5, mélange avec NaCl de la fiole V7 dans la chambre de mélange M4, positionnée en aval de la chambre de réaction R4, et injection dans la chambre de formulation FC, évacuation d'une partie par le port déchet WP ; ouverture des vannes 27, 29, 30, 33 et 34 ; fermeture des vannes 26 et 28 ;
Etape 22-23 (figure 5.14) : remplissage d'une seringue pour un patient via le port seringue SP par du contenu de la chambre de formulation FC, à cet effet injection de gaz par le port gaz GP10 ; ouverture de la vanne 32 ; fermeture des vanne 27, 29, 30, 33 et 34 ; un filtre est présent hors de la carte ; et de même pour un remplissage d'une seringue pour un contrôle qualité ;
Etape 24 (figure 5.15) : séchage du filtre positionné hors de la carte par du gaz introduit par le port GP10 ;
Etape 25 (figure 5.16) : mouillage du filtre par de l'eau déminéralisée de la fiole V4 ; ouverture de la vanne 31 et fermeture de la vanne 32 ; et
Etape 32 (figure 5.17) : réalisation d'un test à point de bulles ; ouverture de la vanne 32 et fermeture de la vanne 31.

## Revendications

1. Cassette microfluidique de synthèse d'un radio-traceur comportant :
- Une carte de support,
- Un circuit microfluidique, intégré au moins en partie dans la carte de support, comportant :
∘ au moins une prise d'alimentation par une fiole (Pf), configurée pour raccorder une fiole (V) au circuit microfluidique,
∘ au moins un port isotope (IP), configuré pour introduire un radio-isotope dans le circuit microfluidique,
∘ au moins une chambre de réaction (R1, R2, R3, R4), raccordée à l'au moins une prise d'alimentation par une fiole et à l'au moins un port isotope par des capillaires,
∘ au moins une chambre de mélange (M1, M2, M3, M4), positionnée en amont de l'au moins une chambre de réaction (R1, R2, R3, R4) et raccordée à l'au moins une chambre de réaction (R1, R2, R3, R4) en amont de laquelle elle est positionnée par au moins un capillaire,
∘ au moins une chambre de formulation (FC), raccordée à l'au moins un port isotope (IP) et à l'au moins une prise d'alimentation par une fiole (Pf) et positionnée en aval de l'au moins une chambre de réaction (R1, R2, R3, R4), et
∘ au moins une prise de connexion pour une seringue (SP), positionnée en aval de l'au moins une chambre de formulation (FC) et raccordée à l'au moins une chambre de formulation par au moins un capillaire.

2. Cassette selon la revendication 1, **caractérisée en ce que** l'au moins une chambre de réaction (R1, R3, R4) est chargée en billes.

3. Cassette selon la revendication 1, **caractérisée en ce que** l'au moins une chambre de réaction est une chambre de réaction en température (R2), et la carte de support comporte un circuit d'isolation thermique entourant au moins une partie de la chambre de réaction en température (R2).

4. Cassette selon la revendication 3, **caractérisée en ce que** le circuit d'isolation thermique comporte au moins un évidement traversant une épaisseur de la carte de support et s'étendant autour d'au moins une partie de la chambre de réaction en température (R2).

5. Cassette selon l'une quelconque des revendications 3 ou 4, **caractérisée en ce que** le circuit microfluidique comporte au moins un évent en amont (Eam) et/ou un évent en aval (Eav) de la chambre de réaction en température (R2) pour évacuer des gaz.

6. Cassette selon l'une quelconque des revendications 1 à 5, **caractérisée en ce que** l'au moins une chambre de mélange (M1, M2, M3, M4) comporte un capillaire et le capillaire comporte une paroi dont au moins une partie comporte une structure en relief.

7. Cassette selon l'une quelconque des revendications 1 à 6, **caractérisée en ce que** le circuit microfluidique comporte au moins une vanne configurée pour ouvrir/fermer un capillaire.

8. Cassette selon l'une quelconque des revendications 1 à 7, **caractérisée en ce qu'**elle comporte un support de fiole fixé sur la carte de support, le support de fiole comportant au moins un emplacement configuré pour recevoir une fiole où débouche l'au moins une prise d'alimentation par une fiole.

9. Procédé de synthèse d'un radio-traceur dans une cassette selon l'une quelconque des revendications précédentes comportant au moins :
- une étape d'injection dans le circuit microfluidique d'un précurseur via l'au moins une prise d'alimentation par une fiole (Pf) et d'un radio-isotope via le port isotope (IP) ;
- une étape de mélange du précurseur et du radio-isotope dans au moins l'une des chambres de mélange (M1, M2, M3, M4) ou des chambres de réaction (R1, R2, R3, R4) ;
- une étape de synthèse du radio-traceur par réaction entre le précurseur et le radio-isotope dans au moins l'une des chambres de réaction (R1, R2, R3, R4) ;
- une étape d'élution du radio-traceur par un solvant injectable à un individu dans au moins l'une des chambres de réaction (R1, R3, R4) ;
- une étape de dilution du radio-traceur dans une solution de NaCl dans la chambre de formulation (FC) ; et
- une étape de remplissage d'une seringue de la solution de NaCl comportant le radio-traceur via le port seringue (SP).

10. Procédé selon la revendication 9 dans lequel l'au moins une étape de réaction comporte une étape de réaction en température dans la chambre de réaction en température (R2).

## Patentansprüche

1. Mikrofluidische Kassette zur Synthese eines radioaktiven Tracers, umfassend:
- eine Trägerplatte,
- einen mikrofluidischen Kreislauf, der mindestens teilweise in die Trägerplatte integriert ist und Folgendes umfasst:
∘ mindestens einen Fläschchen-Einsteckanschluss (Pf), der dazu konfiguriert ist, ein Fläschchen (V) mit dem mikrofluidischen Kreislauf zu verbinden,
∘ mindestens einen Isotopenanschluss (IP), der dazu konfiguriert ist, ein Radioisotop in den mikrofluidischen Kreislauf einzuspeisen,
∘ mindestens eine Reaktionskammer (R1, R2, R3, R4), die mit dem mindestens einen Fläschchen-Einsteckanschluss und mit dem mindestens einen Isotopenanschluss durch Kapillaren verbunden ist,
∘ mindestens eine Mischkammer (M1, M2, M3, M4), die vor der mindestens einen Reaktionskammer (R1, R2, R3, R4) positioniert und mit der mindestens einen Reaktionskammer (R1, R2, R3, R4), vor der sie positioniert ist, durch mindestens eine Kapillare verbunden ist,
∘ mindestens eine Formulierungskammer (FC), die mit dem mindestens einen Isotopenanschluss (IP) und dem mindestens einen Fläschchen-Einsteckanschluss (Pf) verbunden und nach der mindestens einen Reaktionskammer (R1, R2, R3, R4) positioniert ist, und
o mindestens einen Verbindungsanschluss für eine Spritze (SP), der nach der mindestens einen Formulierungskammer (FC) positioniert und mit der mindestens einen Formulierungskammer über mindestens eine Kapillare verbunden ist.

2. Kassette nach Anspruch 1, **dadurch gekennzeichnet, dass** die mindestens eine Reaktionskammer (R1, R3, R4) mit Kügelchen beladen ist.

3. Kassette nach Anspruch 1, **dadurch gekennzeichnet, dass** die mindestens eine Reaktionskammer eine Temperatur-Reaktionskammer (R2) ist und die Trägerplatte einen Wärmeisolationskreislauf umfasst, der mindestens einen Teil der Temperatur-Reaktionskammer (R2) umgibt.

4. Kassette nach Anspruch 3, **dadurch gekennzeichnet, dass** der Wärmeisolationskreislauf mindestens eine Aussparung umfasst, die durch eine Dicke der Trägerplatte verläuft und sich um mindestens einen Teil der Temperatur-Reaktionskammer (R2) erstreckt.

5. Kassette nach einem der Ansprüche 3 oder 4, **dadurch gekennzeichnet, dass** der mikrofluidische Kreislauf mindestens eine Entlüftungsöffnung vor (Eam) und/oder mindestens eine Entlüftungsöffnung nach (Eav) der Temperatur-Reaktionskammer (R2) umfasst, um Gase abzuleiten.

6. Kassette nach einem der Ansprüche 1 bis 5, **dadurch gekennzeichnet, dass** die mindestens eine Mischkammer (M1, M2, M3, M4) eine Kapillare umfasst und die Kapillare eine Wand umfasst, von der mindestens ein Teil eine Reliefstruktur umfasst.

7. Kassette nach einem der Ansprüche 1 bis 6, **dadurch gekennzeichnet, dass** der mikrofluidische Kreislauf mindestens ein Ventil umfasst, das dazu konfiguriert ist, eine Kapillare zu öffnen/schließen.

8. Kassette nach einem der Ansprüche 1 bis 7, **dadurch gekennzeichnet, dass** sie eine Fläschchenhalterung umfasst, die auf der Trägerplatte befestigt ist, wobei die Fläschchenhalterung mindestens eine Aufnahme umfasst, die dazu konfiguriert ist, ein Fläschchen aufzunehmen, wo der mindestens eine Fläschchen-Einsteckanschluss mündet.

9. Verfahren zur Synthese eines radioaktiven Tracers in einer Kassette nach einem der vorhergehenden Ansprüche, mindestens Folgendes umfassend:
- einen Schritt des Injizierens eines Vorläufers über den mindestens einen Fläschchen-Einsteckanschluss (Pf) und eines Radioisotops über den Isotopenanschluss (IP) in den mikrofluidischen Kreislauf;
- einen Schritt des Mischens des Vorläufers und des Radioisotops in mindestens einer der Mischkammern (M1, M2, M3, M4) oder der Reaktionskammern (R1, R2, R3, R4);
- einen Schritt des Synthetisierens des radioaktiven Tracers durch Reaktion zwischen dem Vorläufer und dem Radioisotop in mindestens einer der Reaktionskammern (R1, R2, R3, R4);
- einen Schritt des Eluierens des radioaktiven Tracers durch ein Lösungsmittel, das einer Person injiziert werden kann, in mindestens einer der Reaktionskammern (R1, R3, R4);
- einen Schritt des Verdünnens des radioaktiven Tracers in einer NaCl-Lösung in der Formulierungskammer (FC); und
- einen Schritt des Befüllens einer Spritze mit NaCl-Lösung, die den radioaktiven Tracer enthält, über den Spritzenanschluss (SP).

10. Verfahren nach Anspruch 9, wobei der mindestens eine Reaktionsschritt einen Schritt des Reagierens bei einer Temperatur in der Temperatur-Reaktionskammer (R2) umfasst.

## Claims

1. A microfluidic cassette for synthesizing a radioactive tracer comprising:
- A mounting card,
- A microfluidic circuit, at least partly integrated into the mounting card, comprising:
∘ at least one connector for supply by a vial (Pf), configured to connect a vial (V) to the microfluidic circuit,
∘ at least one isotope port (IP), configured for introducing a radioisotope into the microfluidic circuit,
∘ at least one reaction chamber (R1, R2, R3, R4), connected to the at least one connector for supply by a vial and to the at least one isotope port by capillaries,
∘ at least one mixing chamber (M1, M2, M3, M4), positioned upstream of the at least one reaction chamber (R1, R2, R3, R4) and connected to the at least one reaction chamber (R1, R2, R3, R4) upstream of which it is positioned by at least one capillary,
∘ at least one formulation chamber (FC), connected to the at least one isotope port (IP) and to the at least one connector for supply by a vial (Pf) and positioned downstream of the at least one reaction chamber (R1, R2, R3, R4), and
∘ at least one connector for connecting a syringe (SP), positioned downstream of the at least one formulation chamber (FC) and connected to the at least one formulation chamber by at least one capillary.

2. A cassette according to claim 1, **characterized in that** the at least one reaction chamber (R1, R3, R4) is loaded with beads.

3. A cassette according to claim 1, **characterized in that** the at least one reaction chamber is a chamber for reaction at temperature (R2), and the mounting card comprises a heat insulation flow route surrounding at least part of the chamber for reaction at temperature (R2).

4. A cassette according to claim 3, **characterized in that** the heat insulation flow route comprises at least one recess passing through a thickness of the mounting card and extending around at least part of the chamber for reaction at temperature (R2).

5. A cassette according to any one of claims 3 or 4, **characterized in that** the microfluidic circuit comprises at least one vent upstream (Eam) and/or at least one vent downstream (Eav) of the chamber for reaction at temperature (R2) for discharging gases.

6. A cassette according to any one of claims 1 to 5, **characterized in that** the at least one mixing chamber (M1, M2, M3, M4) comprises a capillary and the capillary comprises a wall at least part of which comprises a structure in relief.

7. A cassette according to any one of claims 1 to 6, **characterized in that** the microfluidic circuit comprises at least one valve configured to open/close a capillary.

8. A cassette according to any one of claims 1 to 7, **characterized in that** it comprises a vial mounting on the mounting card, the vial mounting comprising at least one station configured to receive a vial where the at least one connector for supply by a vial enters.

9. A method for synthesizing a radioactive tracer in a cassette according to any one of the preceding claims comprising at least:
- a step of injecting into the microfluidic circuit a precursor via the at least one connector for supply by a vial (Pf) and a radioisotope via the isotope port (IP);
- a step of mixing the precursor and the radioisotope in at least one of the mixing chambers (M1, M2, M3, M4) or reaction chambers (R1, R2, R3, R4);
- a step of synthesizing the radioactive tracer by reaction between the precursor and the radioisotope in at least one of the reaction chambers (R1, R2, R3, R4);
- a step of eluting the radioactive tracer by a solvent injectable into an individual in at least one of the reaction chambers (R1, R3, R4);
- a step of diluting the radioactive tracer in a solution of NaCl in the formulation chamber (FC); and
- a step of filling a syringe with NaCl solution comprising the radioactive tracer via the syringe port (SP).

10. A method according to claim 9, wherein the at least one reacting step comprises a step of reacting at temperature in the chamber for reaction at temperature (R2).
